# EUROPEAN PATENT APPLICATION

(11) **EP 3 653 608 A1**
(43) Date of publication of application: **20.05.2020**
(21) Application number: 18207030.0
(22) Date of filing: 19.11.2018
(51) Int. Cl.: C07D 217/16, A61K 31/472, A61P 1/16, A61P 9/10, A61P 11/00, A61P 17/06, A61P 35/00, A61P 27/02

(54) **ISOQUINOLINE BIARYL COMPOUNDS, A PHARMACEUTICAL COMPOSITION COMPRISING THE SAME, AND USES THEREOF**

(71) Applicant: Ludwig-Maximilians-Universität München, 80539 München (DE)
(72) Inventor: Thorn-Seshold, Oliver, 80539 München (DE); Bracher, Franz, 82134 Gröbenzell (DE); Melzer, Benedikt, 33602 Bielefeld (DE)
(74) Representative: Vossius & Partner Patentanwälte Rechtsanwälte mbB

(57) **Abstract**

The present invention provides a compound having the formula (I) 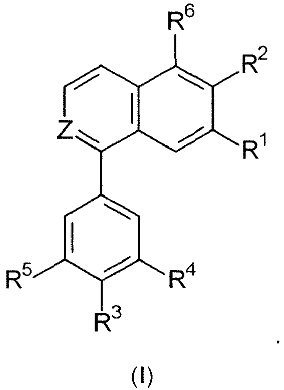 which is suitable as a tubulin polymerization inhibitor and/or an antimitotic cytotoxin. Thus, it can be employed in the treatment, amelioration or prevention of a disorder selected from a neoplastic disorder, atherosclerosis, psoriasis, restenosis, idiopathic pulmonary fibrosis, scleroderma, wet age-related macular degeneration, and cirrhosis of the liver.

## Description

### Technical Field

The present invention relates to isoquinoline biaryl compounds, a pharmaceutical composition comprising the same, their use as a tubulin polymerization inhibitor and/or antimitotic cytotoxin and their use in the treatment, amelioration or prevention of disorders associated therewith.

### Prior Art

A large number of human and veterinary disorders originate from processes of uncontrolled or abnormally high cellular proliferation.

The most important of these disorders is cancer, which is a broad term a group of disorders involving abnormal cell growth with the potential to invade or spread to other parts of the body. Another common disorder related to cellular proliferation is psoriasis. Psoriasis is a long-lasting autoimmune disease characterized by patches of abnormal skin and is characterized by the presence of dry scales and plaques. The disease results from hyperproliferation of the epidermis and incomplete differentiation of keratinocytes. Another disease which is related to cellular proliferation is restenosis. Restenosis is a common adverse event of endovascular procedures. Surgery to widen or unblock a blood vessel usually has a long-lasting beneficial effect for the patient. However, in some cases, the procedure itself can cause further narrowing of the vessel, or restenosis. To widen or unblock a vessel, a stent can be implanted. A stent is a mesh tube-like structure often used in conjunction with angioplasty to permanently hold open an artery, allowing for unrestricted blood flow, or to support a weakness in the artery wall called an aneurysm. The artery can react to the stent, perceive it as a foreign body, and respond by mounting an immune system response which leads to further narrowing near or inside the stent. Also, atherosclerosis, in which the inside of an artery narrows due to the build-up of plaque, can be attributed to cellular proliferation. Furthermore, inflammatory disease states, where endothelial cells, inflammatory cells and glomerular cells are involved; myocardial infarction, where heart muscle cells are involved; glomerular nephritis, where kidney cells are involved; transplant rejection, where endothelial cells are involved; and infectious diseases such as HIV infection and malaria, where certain immune cells and/ or other infected cells are involved, can be related to cellular proliferation.

Abnormally high cellular proliferation can be inhibited by several active agents, which include, amongst others, alkylating agents, topoisomerase inhibitors, nucleotide analogues, antibiotics, hormone antagonists, or nucleic acid damaging agents. One particularly important class of agents are tubulin inhibitors, drugs that bind to the protein tubulin and so affect the dynamics, stability, and function of microtubules. Tubulin is a protein superfamily of globular proteins; notably, α- and β-tubulins copolymerize into microtubules, which are a major component of the eukaryotic cytoskeleton. Microtubules are not static structures but are highly dynamic polymers that are continuously growing and shortening by reversible association and dissociation of α/β-tubulin heterodimers. This dynamic behavior and resulting control over the length and structure of the microtubules is vital to the proper functioning of the microtubule cytoskeleton, and is required for many essential cellular processes, including cell motion, intracellular organelle transport, and mitosis. For example, during the G2/M phase of the cell cycle, microtubules form the mitotic spindle that dynamically reorganizes to facilitate the segregation of sister chromatids and complete mitosis; inhibitors of microtubule polymerization or depolymerization prevent the correct dynamics of this microtubule reorganisation, and can lead to mitotic arrest and death in proliferating cells. In general, the potential to inhibit these essential cellular processes renders the regulation of microtubule dynamics an important target for drug development and is the basis of the therapeutic value of tubulin inhibitors, which find many clinical applications - such as treating gout with colchicine, restenosis with paclitaxel, cancer with paclitaxel, vinblastine, epothilones and halichondrins, and fungal infections with benomyl and malaria and helminths with mebendazole.

Three main classes of tubulin-binding drugs, namely colchicine analogues, Vinca alkaloids, and the taxanes, have been identified, each of which possesses a specific binding site on the β-tubulin molecule. Taxanes such as paclitaxel (Taxol™) stabilize and "freeze" microtubule structures so that they cannot be depolymerized and restructured. Colchicine analogues prevent tubulin polymerization and so disrupt microtubule formation. Vinca alkaloids, including vincristine, and vinblastine prevent microtubule formation and destabilize microtubules. Each of the three classes also includes drugs with relatively unrelated molecular structures but which bind at the same binding site on tubulin and cause similar biological effects, for example dolastatin which binds similarly to Vinca alkaloids, epothilone which binds similarly to taxanes, and mebendazole which binds similarly to colchicine analogues.

Tubulin inhibitors generated particular interest after the introduction of the taxanes and vinca alkaloids into clinical oncology. Compounds targeting the colchicine binding site on tubulin, called colchicine domain inhibitors ("CDIs"), include several advanced drug candidates such as stilbene combretastatins CA4P, CA1P and AVE8062, as well as non-stilbenes BNC105P and ABT751 (see below). CDIs offer a biological activity profile which includes not only the antimitotic and pro-apoptotic effects common to all tubulin polymerisation inhibitors, but also vascular disrupting effects that are considered highly desirable particularly for cancer treatment. This has stimulated much research and development of CDIs and several have reached late-stage clinical trials for cancer treatment.

Despite the discovery of potent CDIs with low or sub-nanomolar antiproliferative activity *in cellulo,* so far no CDIs have been approved for cancer treatment. This can in a large degree be attributed to problems stemming from their spontaneous deactivation *in vivo,* as well as their ADME-PK, and their ease and flexibility of synthetic access.

During drug discovery and medicinal chemistry research, a set of phenyl-chromene biaryls exemplified by compound **mHA1** were reported to bind to tubulin (J. Xi et al.; Molecular Cancer Research 2013, 11, 856-864), and a series of 1-phenyl-naphthyl biaryls exemplified by compound **PNB1** were isolated from plant extracts (J.-L. Cao et al.; Asian J. Chem. 2010, 22, 6509-6512). However, among other problems, (i) the phenyl-chromene scaffold of Xi *et* al. does not allow synthesis of a range of substitution patterns but essentially dictates the substituent pattern because of the chromene synthesis, and (ii) the 1-phenyl-naphthyl biaryls of Cao *et al.* were not investigated for their biochemical target, and were only assayed for anti-HIV activity, leaving the mechanism of action of these compounds unclarified. Various spasmolytically active 3-methyl isoquinolines were also reported (V. Bruckner, G. von Fodor; Ber. dtsch. Chem. Ges. A/B 1938, 71, 541-549), but these were researched as part of efforts towards papverine analogues (phosphodiesterase inhibitors) and their biological mechanism was also not clarified. Further, a series of triaryl compounds exemplified by compound **PIIT1** have also been prepared with the goal to minimise metabolic liabilities of ketone-bridged CDIs (such as **phenstatin**) and these were reported to bind tubulin (D.-J. Hwang et al.; ACS Med. Chem. Lett. 2015, 6, 993-997; Y. Lu et al.; J. Med. Chem. 2014, 57, 7355-7366).

However, (i) those triaryl designs introduce much synthetic complexity as well as molecular weight; and (ii) these triaryls do not exhibit favourable enough solubility (typically, above 10 mM solubility in aqueous media) to enable application *in vivo;* which are unfavourable aspects for pharmaceutical development.

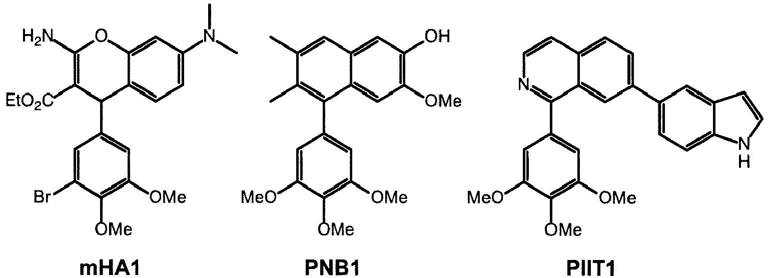

Some specific isoquinoline derivatives related to podophyllotoxin were synthesized (W. Reeve, W.M. Eareckson; J. Am. Chem. Soc. 1950, 72, 5195-5197). However, their potential pharmacological effects were not investigated: Reeve et al. merely assumed that the prepared di- and tetrahydrosubstituted isoquinolines, which have certain of the structural features of podophyllotoxin, might also possess the tumor-damaging properties of podophyllotoxin.

In addition, a series of specific isoquinolines were synthesized by Hati et al., however a possible medical use was not contemplated (S. Hati, S. Sen; Eur. J. Org. Chem. 2017, 1277-1280).

US 2007/128468 discloses certain red phosphorescent compounds and organic electroluminescent devices using the same, which are based on metal complexes containing polymethylated isoquinoline biaryl ligands, however the ligands themselves were not designed to display, nor assayed for, bioactivity.

It was an object of the present invention to provide compounds which are suitable as a tubulin polymerization inhibitor and/or an antimitotic cytotoxin. It was a further object of the present invention to provide compounds which are suitable for treating, ameliorating or preventing a disorder selected from a neoplastic disorder, atherosclerosis, psoriasis, restenosis, idiopathic pulmonary fibrosis, scleroderma, wet age-related macular degeneration, and cirrhosis of the liver, particularly cancer.

### Summary of the Invention

The present invention relates to the following items:
1. A compound having the formula (I) 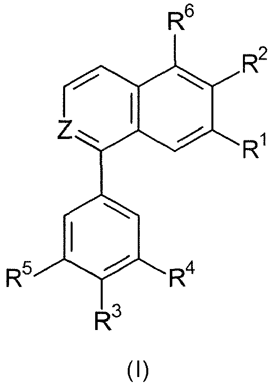 wherein
   - **Z**: is selected from N and N⁺-R^{a}X;
   - **X**: is a counterion;
   - **R¹**: is selected from -C₁₋₄-alkyl, -O-(C₁₋₄-alkyl), -S-(C₁₋₄-alkyl), and -N(R^{b})(R^{c});
   - **R²**: is selected from -H, -OH, -SH, -NH(R^{d}), -halogen, -CH₃, and -OPO₃(R^{e})(R^{f}), or R¹ and R² can be linked together to form -OCH₂O-,
   - **R³**: is selected from -C₁₋₄-alkyl, -OH, -O-(C₁₋₄-alkyl), -SH, -S-(C₁₋₄-alkyl), -halogen, and -OPO₃(R^{e})(R^{f}), wherein -C₁₋₄-alkyl, -O-(C₁₋₄-alkyl) and -S-(C₁₋₄-alkyl) can be optionally substituted by a substituent A;
   - **R⁴**: is selected from -C₁₋₄-alkyl, -O-(C₁₋₄-alkyl), and -S-(C₁₋₄-alkyl), wherein -C₁₋₄-alkyl, -O-(C₁₋₄-alkyl) and -S-(C₁₋₄-alkyl) can be optionally substituted by a substituent A;
   or R³ and R⁴ can be linked together to form -OCH₂O- or -OCH₂CH₂O-;
   - **R⁵**: is selected from -C₁₋₄-alkyl, -O-(C₁₋₄-alkyl), and -S-(C₁₋₄-alkyl), wherein -C₁₋₄-alkyl, -O-(C₁₋₄-alkyl) and -S-(C₁₋₄-alkyl) can be optionally substituted by a substituent A;
   - **R⁶**: is selected from -H, -OH, -SH, -NH(R^{d}), -halogen, -CH₃, and -OPO₃(R^{e})(R^{f});
   - **R^{a}**: is selected from -H and -C₁₋₄-alkyl;
   - **R^{b}**: is selected from -C₁₋₄-alkyl and -proteinogenic amino acids;
   - **R^{c}**: is selected from -H and -C₁₋₄-alkyl;
   - **R^{d}**: is selected from -H, -C₁₋₄-alkyl, and -proteinogenic amino acids;
   - **R^{e}** and **R^{f}**: are independently selected from H, ammonium, alkali ion or alkaline earth metal ion;
   - **R^{g}**: is selected from -H and -C₁₋₄-alkyl;
   - **R^{h}**: is selected from -H and -C₁₋₄-alkyl; and
   - **A**: is selected from -OH, -SH, -N(R^{g})(R^{h}), azetidinyl, and -halogen; wherein azetidinyl is attached to the -C₁₋₄-alkyl, -O-(C₁₋₄-alkyl), or -S-(C₁₋₄-alkyl) group bearing it via the N atom;
   provided that no more than one of the groups **R¹, R², R³, R⁴,** and **R⁵** may simultaneously be -C₁₋₄-alkyl;
   or a pharmaceutically acceptable salt, solvate, or ester thereof.
2. The compound according to item 1, wherein **R¹** and **R²** are linked together to form -OCH₂O-.
3. The compound according to item 1, wherein **R¹** is selected from -C₁₋₄-alkyl, -O-(C₁₋₄-alkyl), and -S-(C₁₋₄-alkyl); preferably wherein **R¹** is -O-(C₁₋₄-alkyl).
4. The compound according to item 1 or 3, wherein **R²** is selected from -H, -OH, -NH(**R^{d}**), and -OPO₃(**R^{e}**)(**R^{f}**), preferably wherein **R²** is selected from -NH(**R^{d}**) and -OPO₃(**R^{e}**)(**R^{f}**).
5. The compound according to any one of items 1 to 4, wherein **R³** is selected from -C₁₋₄-alkyl, -OH, -O-(C₁₋₄-alkyl), and -OPO₃(**R^{e}**)(**R^{f}**), preferably wherein **R³** is -O-(C₁₋₄-alkyl).
6. The compound according to any one of items 1 to 5, wherein **R⁴** is selected from -C₁₋₄-alkyl and -O-(C₁₋₄-alkyl) and/or **R⁵** is selected from -C₁₋₄-alkyl and -O-(C₁₋₄-alkyl).
7. The compound according to any one of items 1 to 6, wherein **R⁶** is selected from -H, -OH, -NH(**R^{d}**), and -OPO₃(**R^{e}**)(**R^{f}**), preferably wherein **R⁶** is selected from -OH and -OPO₃(**R^{e}**)(**R^{f}**).
8. The compound according to any one of items 1 to 7, wherein **Z** is N.
9. The compound according to any one of items 1 to 7, wherein **Z** is N⁺-R^{a}X, preferably wherein **R^{a}** is -C₁₋₄-alkyl.
10. The compound according to any one of items 1 to 9, wherein no more than two of R¹, R³, R⁴ and R⁵ are simultaneously -C₁₋₄-alkyl.
11. The compound according to item 8, wherein R¹ is selected from -Me, -Et, -OMe, -OEt, -SMe and -SEt.
12. The compound according to item 8, wherein R³ is selected from -Me, -Et, -OH, -OMe, -OEt, -OPO₃H₂, and -OPO₃Na₂.
13. The compound according to item 8, wherein R⁴ is selected from -Me, -Et, -OMe, and -OEt; and R⁵ is selected from -Me, -Et, -OMe, and -OEt.
14. The compound according to item 8, wherein R² is selected from -H, -OH, -NH₂, -NH(proteinogenic amino acid), -OPO₃H₂, and -OPO₃Na₂; and R⁶ is selected from -H, -OH, -NH₂ -OPO₃H₂, and -OPO₃Na₂.
15. The compound according to item 8, wherein R¹ is selected from -Me, -Et, -OMe, -OEt, -SMe and -SEt; R² is selected from -OH, -NH₂, -NH(proteinogenic amino acid), -OPO₃H₂, and -OPO₃Na₂; R³ is selected from -Me, -Et, -OMe, and -OEt; R⁴ is selected from -Me, -Et, -OMe, and -OEt; R⁵ is selected from -Me, -Et, -OMe, and -OEt; and R⁶ is selected from -H, -OH, -NH₂, -OPO₃H₂, and -OPO₃Na₂.
16. The compound according to item 15, wherein R¹ is OMe; R² is selected from -OH, -NH₂, -NH(proteinogenic amino acid), -OPO₃Na₂ and -OPO₃H₂; R³ is selected from -Et and -OMe; R⁴ is selected from -Et and -OMe; and R⁵ is selected from -Et and -OMe.
17. The compound according to item 1, wherein **Z** is N⁺-R^{a}X and Rₐ is selected from Me, Et, and n-Pr.
18. The compound according to item 17, wherein R¹ is selected from -Me, -Et, -OMe, -OEt, -SMe and -SEt; R² is selected from -H, -OH, -NH₂, -NH(proteinogenic amino acid), -OPO₃H₂, and -OPO₃Na₂; R³ is selected from -Me, -Et, -OH, -OMe, -OEt, -OPO₃H₂, and -OPO₃Na₂; R⁴ is selected from -Me, -Et, -OMe, and -OEt; R⁵ is selected from -Me, -Et, -OMe, and -OEt; and R⁶ is selected from -H, -OH, -NH₂, -OPO₃H₂, and -OPO₃Na₂.
19. The compound according to item 18, wherein R¹ is OMe; R² is selected from -OH, -NH₂, -NH(proteinogenic amino acid), -OPO₃Na₂ and -OPO₃H₂; R³ is selected from -Me, -Et, -OH, -OMe, -OEt, -OPO₃H₂, and -OPO₃Na₂; R⁴ is selected from -Et and -OMe; and R⁵ is selected from -Et and -OMe.
20. A pharmaceutical composition comprising a compound according to any one of items 1 to 19, or a pharmaceutically acceptable salt, solvate, or ester thereof, and optionally a pharmaceutically acceptable carrier.
21. A compound according to any one of items 1 to 19, or a pharmaceutically acceptable salt, solvate, or ester thereof, for use as a tubulin polymerization inhibitor and/or an antimitotic cytotoxin.
22. A compound according to any one of items 1 to 19, or a pharmaceutically acceptable salt, solvate, or ester thereof, for use in the treatment, amelioration or prevention of a disorder selected from a neoplastic disorder, atherosclerosis, psoriasis, restenosis, idiopathic pulmonary fibrosis, scleroderma, wet age-related macular degeneration, and cirrhosis of the liver.
23. The compound for use according to item 22, wherein the disorder is a neoplastic disorder, in particular cancer.
24. The compound for use according to item 23, wherein the cancer is selected from leukemias including acute myeloid leukemia, myelodysplastic syndrome, fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon carcinoma, pancreatic cancer, breast cancer, ovarian cancer, prostate cancer, squamous cell carcinoma, basal cell carcinoma, adenocarcinoma, hepatocellular carcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilms' tumor, cervical cancer, testicular tumor, lung carcinoma, small cell lung carcinoma, non-small cell lung carcinoma, bladder carcinoma, epithelial carcinoma, glioblastoma, glioma, astrocytoma, medulloblastoma, craniopharyngioma, ependymoma, pinealoma, hemangioblastoma, acoustic neuroma, oligodendroglioma, meningioma, melanoma, neuroblastoma, and retinoblastoma.
25. Use of a compound according to any one of items 1 to 19, or a pharmaceutically acceptable salt, solvate, or ester thereof, for the manufacture of a tubulin polymerization inhibitor and/or an antimitotic cytotoxin.
26. Use of a compound according to any one of items 1 to 19, or a pharmaceutically acceptable salt, solvate, or ester thereof, for the manufacture of a medicament for treating, ameliorating or preventing of a disorder selected from a neoplastic disorder, atherosclerosis, psoriasis, restenosis, idiopathic pulmonary fibrosis, scleroderma, wet age-related macular degeneration, and cirrhosis of the liver.
27. The use according to item 26, wherein the disorder is a neoplastic disorder, in particular cancer.
28. The use according to item 27, wherein the cancer is selected from leukemias including acute myeloid leukemia, myelodysplastic syndrome, fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon carcinoma, pancreatic cancer, breast cancer, ovarian cancer, prostate cancer, squamous cell carcinoma, basal cell carcinoma, adenocarcinoma, hepatocellular carcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilms' tumor, cervical cancer, testicular tumor, lung carcinoma, small cell lung carcinoma, non-small cell lung carcinoma, bladder carcinoma, epithelial carcinoma, glioblastoma, glioma, astrocytoma, medulloblastoma, craniopharyngioma, ependymoma, pinealoma, hemangioblastoma, acoustic neuroma, oligodendroglioma, meningioma, melanoma, neuroblastoma, and retinoblastoma.
29. A method of treating, ameliorating or preventing a disorder associated with tubulin polymerization, wherein an effective amount of a compound according to any one of items 1 to 19, or a pharmaceutically acceptable salt, solvate, or ester thereof, is administered to a patient in need thereof.
30. A method of treating, ameliorating or preventing a disorder selected from a neoplastic disorder, atherosclerosis, psoriasis, restenosis, idiopathic pulmonary fibrosis, scleroderma, wet age-related macular degeneration, and cirrhosis of the liver, wherein an effective amount of a compound according to any one of items 1 to 19, or a pharmaceutically acceptable salt, solvate, or ester thereof, is administered to a patient in need thereof.
31. The method according to item 29 or 30, wherein the disorder is a neoplastic disorder, in particular cancer.
32. The method according to item 31, wherein the cancer is selected from leukemias including acute myeloid leukemia, myelodysplastic syndrome, fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon carcinoma, pancreatic cancer, breast cancer, ovarian cancer, prostate cancer, squamous cell carcinoma, basal cell carcinoma, adenocarcinoma, hepatocellular carcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilms' tumor, cervical cancer, testicular tumor, lung carcinoma, small cell lung carcinoma, non-small cell lung carcinoma, bladder carcinoma, epithelial carcinoma, glioblastoma, glioma, astrocytoma, medulloblastoma, craniopharyngioma, ependymoma, pinealoma, hemangioblastoma, acoustic neuroma, oligodendroglioma, meningioma, melanoma, neuroblastoma, and retinoblastoma.

One or more of the following compounds as well as pharmaceutically acceptable salts and solvates thereof can be excluded from one or more of the above items:

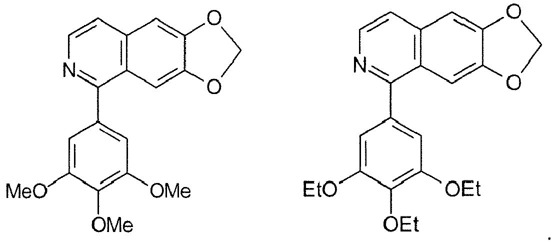

These compounds are 5-(3,4,5-trimethoxyphenyl)-[1,3]dioxolo[4,5-*g*]isoquinoline (W. Reeve, W.M. Eareckson; J. Am. Chem. Soc. 1950, 72, 5195-5197) and 5-(3,4,5-triethoxyphenyl)-[1,3]dioxolo[4,5-*g*]isoquinoline (Patent DE 614703), respectively.

### Description of the figures

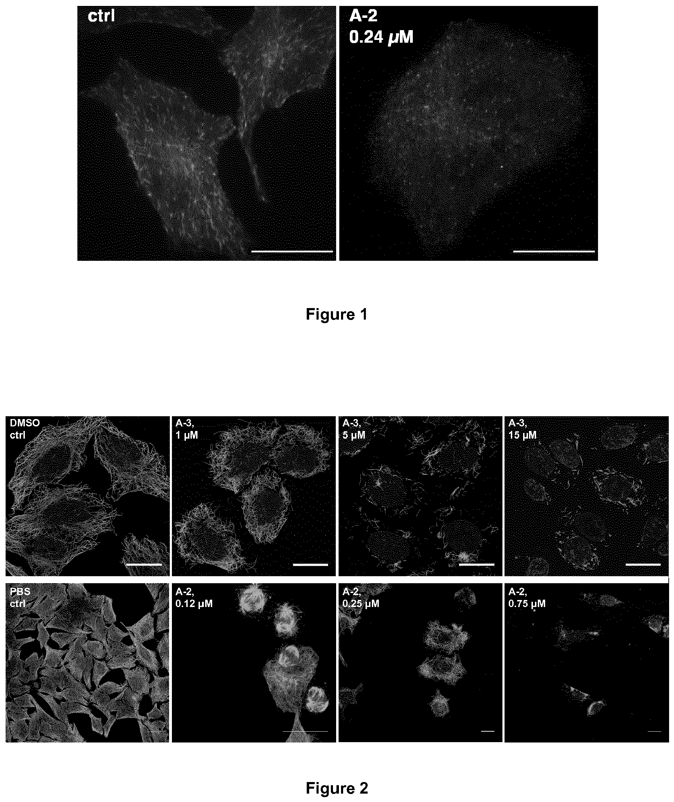

Figure 1 shows still frames from confocal microscopy video imaging of live HeLa cells transfected to express EB3-YFP fusion protein, a fluorescent marker that selectively labels the growing tips of microtubules (MTs). In videos this marker manifests as bright well-defined moving "comets", and in still images as bright elongated streaks, when MTs are polymerising normally as in the control image (frame "ctrl"). Upon inhibition of MT polymerisation the comets stop, fade, and disappear, which manifests as dim points in still images (M. Borowiak et al.; Cell 2015, 162, 403-411). After applying **A-2** at 0.24 µM the comets stopped and vanished within 0.5 minutes of treatment, showing that **A-2** causes potent inhibition of tubulin polymerisation dynamics (frame "**A-2** 0.24 µM").
Figure 2 shows confocal microscopy images of the cellular microtubule network, labeled by immunofluorescent staining for tubulin (light grey), with the nucleus counterstained by DAPI (dark grey), according to published protocols (M. Borowiak et al.; Cell 2015, 162, 403-411). Images were acquired after 24 h treatment with compounds **A-2** (administered in PBS) or **A-3** (administered with 0.25% DMSO cosolvent). "DMSO ctrl" denotes the cosolvent control for the **A-3** images (i.e. 0.25% DMSO but no compound) while "PBS ctrl" denotes the control for the **A-2** images (no compound or cosolvent applied); these controls show the expected structure of the microtubule network when no inhibition is applied. The pronounced disorganisation and destruction of the microtubule network observed with increasing concentrations of compounds **A-2** and **A-3** is only expected for the most potent members of the CDI class.

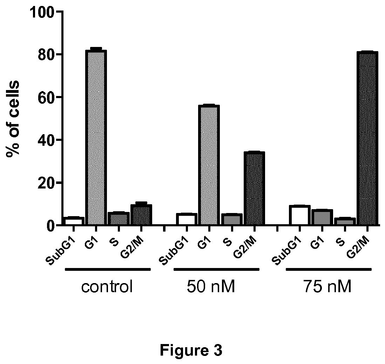

Figure 3 shows results of cell cycle analysis of HeLa cells incubated with **A-2** for 24 h, which were analysed by flow cytometry after staining for DNA with propidium iodide by standard protocols (M. Borowiak et al.; Cell 2015, 162, 403-411). These results show extensive and dose-dependent perturbation of the typical cell cycle ("control" results, obtained without compound administration), since **A-2** administration dose-dependently causes accumulation of cells at the G2/M phase of the cell cycle, which is expected for potent CDIs.

### Definitions

The term "alkyl" refers to a saturated straight or branched hydrocarbon chain, which has 1 to 4 carbon atoms, preferably 1 to 3 carbon atoms, more preferably 1 or 2 carbon atoms, even more preferably 1 carbon atom. In any of the below definitions, the C₁₋₄-alkyl in -C₁₋₄-alkyl, -O-(C₁₋₄-alkyl), -S-(C₁₋₄-alkyl) is not particularly limited, preferably C₁₋₄-alkyl can be methyl, ethyl, n-propyl, or i-propyl, more preferably it can be methyl or ethyl, most preferably it can be methyl.

The term "halogen" covers F, Cl, Br, and I, preferably chloride.

If a compound or moiety is referred to as being "optionally substituted by a substituent A" it can in each instance include one or more of the indicated substituents A, whereby the substituents A can be the same or different.

The term "pharmaceutically acceptable salt" refers to a salt of a compound of the present invention. Suitable pharmaceutically acceptable salts include acid addition salts which may, for example, be formed by mixing a solution of compounds of the present invention with a solution of a pharmaceutically acceptable acid such as hydrochloric acid, sulfuric acid, fumaric acid, maleic acid, succinic acid, acetic acid, benzoic acid, citric acid, tartaric acid, carbonic acid or phosphoric acid. Furthermore, where the compound carries an acidic moiety, suitable pharmaceutically acceptable salts thereof may include alkali metal salts (e.g., sodium or potassium salts); alkaline earth metal salts (e.g., calcium or magnesium salts); and salts formed with suitable organic ligands (e.g., ammonium, quaternary ammonium and amine cations formed using counteranions such as halide, hydroxide, carboxylate, sulfate, phosphate, nitrate, alkyl sulfonate and aryl sulfonate). Illustrative examples of pharmaceutically acceptable salts include, but are not limited to, acetate, adipate, alginate, ascorbate, aspartate, benzenesulfonate, benzoate, bicarbonate, bisulfate, bitartrate, borate, bromide, butyrate, calcium edetate, camphorate, camphorsulfonate, camsylate, carbonate, chloride, citrate, clavulanate, cyclopentanepropionate, digluconate, dihydrochloride, dodecylsulfate, edetate, edisylate, estolate, esylate, ethanesulfonate, formate, fumarate, gluceptate, glucoheptonate, gluconate, glutamate, glycerophosphate, glycolylarsanilate, hemisulfate, heptanoate, hexanoate, hexylresorcinate, hydrabamine, hydrobromide, hydrochloride, hydroiodide, 2-hydroxy-ethanesulfonate, hydroxynaphthoate, iodide, isothionate, lactate, lactobionate, laurate, lauryl sulfate, malate, maleate, malonate, mandelate, mesylate, methanesulfonate, methylsulfate, mucate, 2-naphthalenesulfonate, napsylate, nicotinate, nitrate, N-methylglucamine ammonium salt, oleate, oxalate, pamoate (embonate), palmitate, pantothenate, pectinate, persulfate, 3-phenylpropionate, phosphate/diphosphate, picrate, pivalate, polygalacturonate, propionate, salicylate, stearate, sulfate, succinate, tannate, tartrate, teoclate, tosylate, triethiodide, undecanoate, valerate, and the like (see, for example, S. M. Berge et al.; J. Pharm. Sci. 1977, 66, 1-19).

When the compounds of the present invention are provided in crystalline form, the structure can contain solvent molecules. The solvents are typically pharmaceutically acceptable solvents and include, among others, water (hydrates) or organic solvents. Examples of possible solvates include ethanolates and iso-propanolates.

The term "pharmaceutically acceptable ester" refers to an ester of a compound of the present invention. Suitable pharmaceutically acceptable esters include acetyl, butyryl, and acetoxymethyl esters.

The term "proteinogenic amino acids" here comprises the 20 principal naturally-occurring L-amino acids (glycine, alanine, valine, leucine, isoleucine, phenylalanine, proline, methionine, serine, threonine, cysteine, tyrosine, tryptophan, asparagic acid, glutamic acid, asparagine, arginine, histidine, lysine, asparagine and glutamine) which are to be attached, as the monopeptide, via their carboxyl terminus to the aniline nitrogen of the compound, creating anilides. Preferably the proteinogenic amino acid is selected from leucine, serine and lysine.

The term "tubulin polymerization inhibitor" here refers to compounds (that are also known as microtubule inhibitors) which alter the typical speeds of microtubule polymerisation or depolymerization, typically by directly binding to the protein tubulin which is the constituent unit of microtubules. Such compounds include diverse superfamilies of compounds such as taxanes, Vinca alkaloids, antimitotic cytotoxin, and others.

The term "antimitotic cytotoxin" relates to tubulin polymerisation inhibitors that bind to the protein tubulin at the colchicine binding site. They have been comprehensively reviewed in Y. Lu et al.; Pharmaceutical Research 2012, 29, 2943-2971; and references therein.

### Detailed description of the invention

### Compound having formula (I)

The present invention provides a compound having the formula (I)

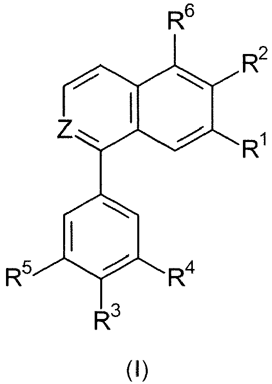

**Z** is selected from N and N⁺-**R^{a}**X. In one embodiment, **Z** is N. In another embodiment, N⁺-**R^{a}**X.

**X** is an anionic counterion. The counterion is not particularly limited, any (particularly pharmaceutically acceptable) counterion can be used in the compound of the present invention. Preferably, the counterion can be selected from the group consisting of halide (e.g., chloride, bromide, iodide), acetate, adipate, alginate, ascorbate, aspartate, benzenesulfonate, benzoate, bicarbonate, bisulfate, bitartrate, borate, butyrate, calcium edetate, camphorate, camphorsulfonate, carbonate, citrate, clavulanate, cyclopentanepropionate, dodecylsulfate, edetate, edisylate, ethanesulfonate, formate, fumarate, glucoheptonate, gluconate, glutamate, glycerophosphate, heptanoate, hexanoate, 2-hydroxyethanesulfonate, hydroxynaphthoate, lactate, lactobionate, laurate, lauryl sulfate, malate, maleate, malonate, mandelate, methanesulfonate, methylsulfate, mucate, 2-naphthalenesulfonate, nicotinate, oleate, oxalate, pamoate (embonate), palmitate, pantothenate, pectinate, 3-phenylpropionate, phosphate/diphosphate, picrate, pivalate, polygalacturonate, propionate, salicylate, stearate, sulfate, succinate, tannate, tartrate, teoclate, tosylate, undecanoate, valerate, more preferably the counterion can be halide, methanesulfonate or tosylate, furthermore preferably chloride or iodide, most preferably the counterion can be chloride.

**R¹** is selected from -C₁₋₄-alkyl, -O-(C₁₋₄-alkyl), -S-(C₁₋₄-alkyl), and -N(**R^{b}**)(**R^{c}**). Preferably **R¹** can be -C₁₋₄-alkyl, -O-(C₁₋₄-alkyl), and -S-(C₁₋₄-alkyl), more preferably **R¹** can be -C₁₋₄-alkyl or -O-(C₁₋₄-alkyl), even more preferably -O-(C₁₋₄-alkyl). Preferably **R¹** can be selected from the group consisting of -CH₃, -CH₂CH₃, -OMe, -OEt, -SMe, -SEt, -NHMe, -NMe₂, -NHEt, -NEt₂, and -NMeEt, more preferably from the group consisting of -Me, -Et, -OMe, -OEt, -SMe and -SEt, even more preferably -Me, -Et, -OMe and -OEt, most preferably **R¹** can be -OMe or -Et or more particularly -OMe.

**R²** is selected from -H, -OH, -SH, -NH(**R^{d}**), -halogen, -CH₃, and -OPO₃(**R^{e}**)(**R^{f}**). Preferably **R²** can be selected from -H, -OH, -NH(**R^{d}**), and -OPO₃(R^{e})(R^{f}), more preferably from -NH(**R^{d}**), and -OPO₃(**R^{e}**)(**R^{f}**). Preferably **R²** can be selected from -H, -OH, -NH₂, -NH(proteinogenic amino acid), -OPO₃H₂, -OPO₃Na₂, even more preferably **R²** can be selected from -NH(-proteinogenic amino acid) and -OPO₃Na₂.

Alternatively, **R¹** and **R²** can be linked together to form -OCH₂O-. When **R¹** and **R²** are linked together to form -OCH₂O-, then preferably either
(i) **R⁶** cannot be H, and/or
(ii) **R³** cannot be -C₁₋₄-alkyl or -O-C₁₋₄-alkyl or -S-C₁₋₄-alkyl, and/or
(iii) **R^{a}** is -C₁₋₄-alkyl
These compounds have improved bioactivity and solubility.

**R³** is selected from -C₁₋₄-alkyl, -OH, -O-(C₁₋₄-alkyl), -SH, -S-(C₁₋₄-alkyl), -halogen (i.e. -F, -CI, -Br, or -I), and -OPO₃(**R^{e}**)(**R^{f}**), wherein -C₁₋₄-alkyl, -O-(C₁₋₄-alkyl) and -S-(C₁₋₄-alkyl) can be optionally substituted by a substituent **A.** Preferably **R³** can be selected from -C₁₋₄-alkyl, -OH, -O-(C₁₋₄-alkyl), and -OPO₃(**R^{e}**)(**R^{f}**), more preferably **R³** can be -O-(C₁₋₄-alkyl), wherein -C₁₋₄-alkyl and -O-(C₁₋₄-alkyl) can be optionally substituted by a substituent **A.** Preferably **R³** can be -Me, -Et -OH, -OMe, -OEt, -SH, -SMe, -SEt -OCH₂CH₂NH₂, -OCH₂CH₂Cl, -OCH₂CH₂OH, -OCH₂CH₂SH, -F, -CI, -OPO₃H₂, or -OPO₃Na₂, more preferably **R³** can be -Me, -Et, -OH, -OMe, -OEt, -OPO₃H₂, or -OPO₃Na₂, even more preferably **R³** can be -Me, -Et, -OMe, and -OEt, furthermore preferably **R³** can be -OMe or -Et, more particularly -OMe.

**R⁴** is selected from -C₁₋₄-alkyl, -O-(C₁₋₄-alkyl), and -S-(C₁₋₄-alkyl), wherein -C₁₋₄-alkyl, -O-(C₁₋₄-alkyl), and -S-(C₁₋₄-alkyl) can be optionally substituted by a substituent **A.** Preferably **R⁴** can be selected from -C₁₋₄-alkyl and -O-(C₁₋₄-alkyl), wherein -C₁₋₄alkyl and -O-(C₁₋₄-alkyl) can be optionally substituted by **A,** more preferably **R⁴** can be selected from -O-C₁₋₄-alkyl, wherein -C₁₋₄-alkyl can be optionally substituted by **A.** Preferably **R⁴** can be selected from -Me, -Et, -OMe, -OEt, -SMe or -SEt, more preferably **R⁴** can be selected from -Me, -Et, -OMe, and -OEt, even more preferably **R⁴** can be -OMe or -Et, particularly -OMe.

In an alternative embodiment, **R³** and **R⁴** can be linked together to form -OCH₂O- or -OCH₂CH₂O-.

**R⁵** is selected from -C₁₋₄-alkyl, -O-(C₁₋₄-alkyl), and -S-(C₁₋₄-alkyl), wherein -C₁₋₄-alkyl, -O-(C₁₋₄-alkyl), and -S-(C₁₋₄-alkyl) can be optionally substituted by a substituent **A.** Preferably **R⁵** can be selected from -C₁₋₄-alkyl and -O-(C₁₋₄-alkyl), wherein -C₁₋₄-alkyl and -O-(C₁₋₄-alkyl) can be optionally substituted by **A,** more preferably **R⁵** can be selected from -O-C₁₋₄-alkyl, wherein -C₁₋₄-alkyl can be optionally substituted by **A.** Preferably **R⁵** can be selected from -Me, -Et, -OMe, -OEt, -SMe or -SEt, more preferably **R⁵** can be selected from -Me, -Et, -OMe, and -OEt, even more preferably **R⁵** can be -OMe or -Et, particularly -OMe.

**R⁶** is selected from -H, -OH, -SH, -NH(**R^{d}**), -halogen, -CH₃, and -OPO₃(**R^{e}**)(**R^{f}**), preferably **R⁶** can be selected from -H, -OH, -NH(**R^{d}**), and -OPO₃(R^{e})(R^{f}) more preferably **R⁶** can be selected from -H, -OH and -OPO₃(**R^{e}**)(**R^{f}**). Preferably **R⁶** can be selected from -H, -OH, -SH, -NH₂, -NH(proteinogenic amino acid), -F, -CH₃, -OPO₃H₂, and -OPO₃Na₂, more preferably from -H, -OH, -NH₂, -OPO₃H₂, and -OPO₃Na₂, and even more preferably from -H, -OH, -OPO₃H₂, and -OPO₃Na₂.

The selections of substituents can be made freely within these possibilities, provided that no more than one of the groups **R¹, R², R³, R⁴,** and **R⁵** may simultaneously be-C₁₋₄-alkyl. This ensures that the compounds obtained are sufficiently bioactive and soluble.

**R^{a}** is selected from -H and -C₁₋₄-alkyl, preferably -H, -methyl, -ethyl, -(*n*-propyl), or -(*i*-propyl), more preferably -H, -methyl or -ethyl, most preferably -H or -methyl.

**R^{b}** is selected from -C₁₋₄-alkyl and -proteinogenic amino acids, preferably -methyl, -ethyl, -(*n*-propyl), -(*i*-propyl) and -proteinogenic amino acids, more preferably -methyl, -ethyl and -proteinogenic amino acids, most preferably -methyl and -proteinogenic amino acids.

**R^{c}** is selected from -H and -C₁₋₄-alkyl, preferably -H, -methyl, -ethyl, -(*n*-propyl) and -(*i*-propyl), more preferably -H, -methyl or -ethyl, most preferably -H and -methyl.

**R^{d}** is selected from -H, -C₁₋₄-alkyl, and -proteinogenic amino acids, preferably -H, -proteinogenic amino acids, -methyl, -ethyl, -(*n*-propyl), and -(*i*-propyl), more preferably -H, -proteinogenic amino acids, -methyl and -ethyl, most preferably -H, -proteinogenic amino acids and -methyl.

**R^{e}** and **R^{f}** are independently selected from H, ammonium, alkali ion or alkaline earth metal ion. The alkali ion is not particularly limited, preferably the alkali ion can be Na or K, more preferably Na. The alkaline earth metal ion is not particularly limited, preferably the alkaline earth metal ion can be Mg or Ca. Preferably **R^{e}** and **R^{f}** can be independently selected from H and Na.

**R^{g}** is selected from -H and C₁₋₄-alkyl, preferably -H, -methyl, -ethyl, -(*n*-propyl) and -(*i*-propyl), more preferably -H, -methyl and -ethyl, most preferably -H and -methyl.

**R^{h}** is selected from -H and C₁₋₄-alkyl, preferably -H, -methyl, -ethyl, -(n-propyl) and -(*i*-propyl), more preferably -H, -methyl and -ethyl, most preferably -H and -methyl.

A is selected from -OH, -SH, -N(**R^{g}**)(**R^{h}**), azetidinyl, and -halogen; wherein azetidinyl is attached to the -C₁₋₄-alkyl, -O-(C₁₋₄-alkyl) or -S-(C₁₋₄-alkyl) group bearing it via the N atom. Preferably **A** can be selected from -OH, -NH₂, and -CI.

In a preferred embodiment, R³, R⁴ and R⁵ are independently -O-(C₁₋₄-alkyl), preferably R³, R⁴ and R⁵ are independently selected from -Me, -Et, -OMe, and -OEt, more preferably R³, R⁴ and R⁵ are independently selected from -Et and -OMe, even more preferably R³, R⁴ and R⁵ are -OMe.

It is understood that all combinations of the above definitions and preferred definitions are envisaged by the present inventors.

Representative compounds include

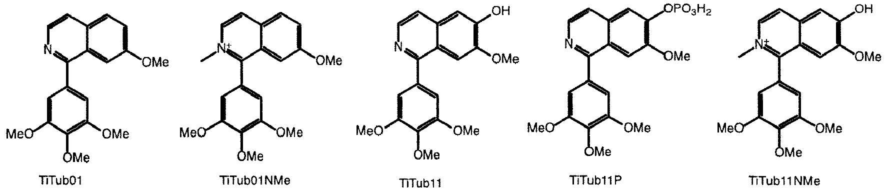

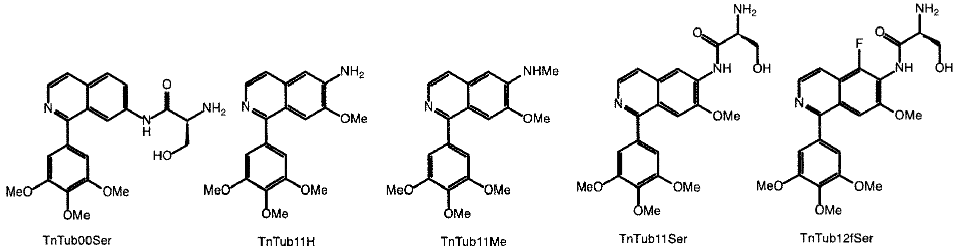

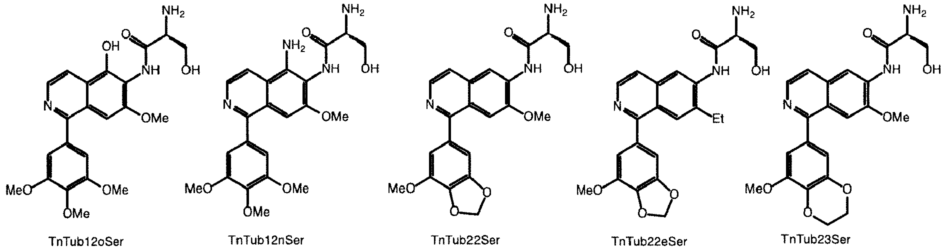

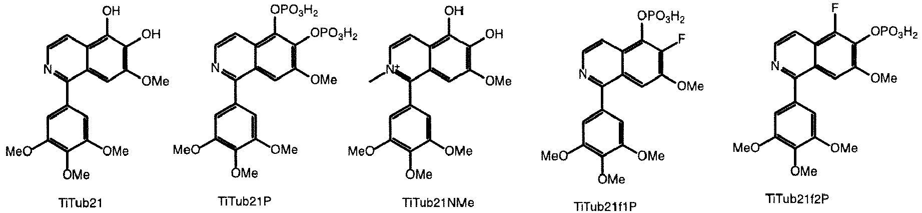

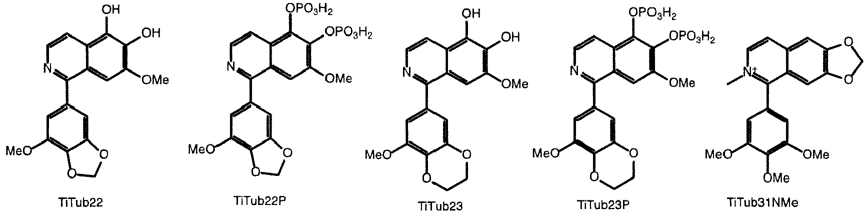

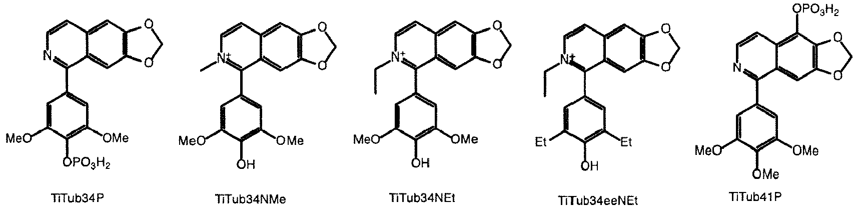

Without wishing to be bound by theory, it is assumed that the phenyl isoquinoline biaryl linkage may offer advantages since C=C linkages in CDI stilbenes such as **CA4P** are known to undergo spontaneous cis-to-trans isomerisation *in vivo* which inactivates the tubulin binding ability of the compound (G. C. Tron et al.; J. Med. Chem. 2006, 49, 3033-3044; D. Tarade et al.; PLoS One 2017, 12, e0171806) and is also a probable site for potent inhibition of and/or metabolism by cytochromes (S. Kim et al.; J. Med. Chem. 2002, 45, 160-164), and since ketone linkages (as in **BNC105P** or **phenstatin)** are also known to be metabolically labile (D.-J. Hwang et al.; ACS Med. Chem. Lett. 2015, 6, 993-997; Y. Lu et al.; J. Med. Chem. 2014, 57, 7355-7366). Biphenyl type biaryls such as the colchicine mimic **MP5-F9** (Tarade et al.; PLoS One 2017, 12, e0171806) have previously been developed but as far as we are aware were unfavourable for clinical development, probably since the potency of these compounds is approximately 500-fold less than of the stilbene-type CDIs.

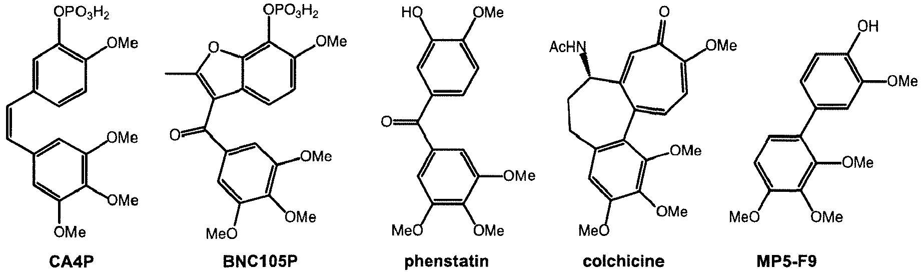

The compounds of the present invention have been shown to possess excellent stability and to be easily accessible by standard synthetic routes. The compounds of the formula (I) retain the high potency of the best current CDIs, while delivering robustness to the spontaneous deactivation mechanism affecting stilbene compounds, avoiding other metabolic deactivation pathways that can affect the current set of CDIs, ensuring simple synthetic access, and offering potentially advantageous ADME-PK compared to current CDIs.

### Synthesis of the compounds having the formula (I)

The compounds having the formula (I) can be prepared by any desired synthetic route. In one illustrative embodiment, which is not limiting, isoquinolines bearing groups R¹, R² and R⁶ (or their protected variants or their synthetic precursor groups) are halogenated at the 1-position eg. using TMPMgCl·LiCl and I₂, then a Suzuki coupling with phenylboronic acids (or phenylboronic esters) bearing groups R³, R⁴ and R⁵ (or their protected variants or their synthetic precursors) delivers isoquinoline biaryl compounds that may be directly compounds of the invention (or which may be compounds of the invention following synthetic modification of the precursor groups or deprotection of protecting groups), and which may also be alkylated eg. by alkyl halides to yield N-alkyl isoquinoliniums that may be directly compounds of the invention (or which may be compounds of the invention following synthetic modification of the precursor groups or deprotection of protecting groups), according to the scheme:

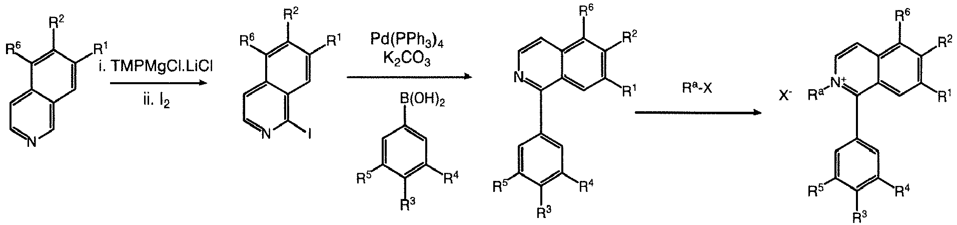

### Pharmaceutical compositions

The compounds of the present invention can be administered to a patient in the form of a pharmaceutical composition which can optionally comprise one or more pharmaceutical carrier(s).The pharmaceutical composition can further optionally contain one or more pharmaceutically excipient(s).

The compounds of the present invention can be administered by various well known routes, including oral, rectal, intragastrical, intracranial and parenteral administration, e.g. intravenous, intramuscular, intranasal, intradermal, subcutaneous, and similar administration routes. Parenteral and oral administration are preferred, particularly preferred is intravenous administration.

Particular preferred pharmaceutical forms for the administration of a compound of the invention are forms suitable for injectable use and include sterile aqueous solutions or dispersions and sterile powders for the extemporaneous preparation of sterile injectable solutions or dispersion. In all cases the final solution or dispersion form must be sterile and fluid. Typically, such a solution or dispersion will include a solvent or dispersion medium, containing, for example, water-buffered aqueous solutions, e.g. biocompatible buffers, ethanol, polyol, such as glycerol, propylene glycol, polyethylene glycol, suitable mixtures thereof, surfactants or vegetable oils. A compound of the invention can also be formulated into liposomes, in particular for parenteral administration. Liposomes provide the advantage of increased half life in the circulation, if compared to the free drug and a prolonged more even release of the enclosed drug.

Sterilization of infusion or injection solutions can be accomplished by any number of art recognized techniques including but not limited to addition of preservatives like anti-bacterial or anti-fungal agents, e.g. parabene, chlorobutanol, phenol, sorbic acid or thimersal. Further, isotonic agents, such as sugars or salts, in particular sodium chloride may be incorporated in infusion or injection solutions.

Production of sterile injectable solutions containing one or several of the compounds of the invention is accomplished by incorporating the respective compound in the required amount in the appropriate solvent with various ingredients enumerated above as required followed by sterilization. To obtain a sterile powder the above solutions are vacuum-dried or freeze-dried as necessary. Preferred diluents of the present invention are water, physiological acceptable buffers, physiological acceptable buffer salt solutions or salt solutions. Preferred carriers are cocoa butter and vitebesole. Excipients which can be used with the various pharmaceutical forms of a compound of the invention can be chosen from the following non-limiting list:
a) binders such as lactose, mannitol, crystalline sorbitol, dibasic phosphates, calcium phosphates, sugars, microcrystalline cellulose, carboxymethyl cellulose, hydroxyethyl cellulose, polyvinyl pyrrolidone and the like;
b) lubricants such as magnesium stearate, talc, calcium stearate, zinc stearate, stearic acid, hydrogenated vegetable oil, leucine, glycerids and sodium stearyl fumarates,
c) disintegrants such as starches, croscaramellose, sodium methyl cellulose, agar, bentonite, alginic acid, carboxymethyl cellulose, polyvinyl pyrrolidone and the like.

In one embodiment the formulation is for oral administration and the formulation comprises one or more or all of the following ingredients: pregelatinized starch, talc, povidone K 30, croscarmellose sodium, sodium stearyl fumarate, gelatin, titanium dioxide, sorbitol, monosodium citrate, xanthan gum, titanium dioxide, flavoring, sodium benzoate and saccharin sodium.

Other suitable excipients can be found in the Handbook of Pharmaceutical Excipients, published by the American Pharmaceutical Association, which is herein incorporated by reference.

It is to be understood that depending on the severity of the disorder and the particular type which is treatable with one of the compounds of the invention, as well as on the respective patient to be treated, e.g. the general health status of the patient, etc., different doses of the respective compound are required to elicit a therapeutic or prophylactic effect. The determination of the appropriate dose lies within the discretion of the attending physician. It is contemplated that the dosage of a compound of the invention in the therapeutic or prophylactic use of the invention should be in the range of about 30 mg to about 200 mg of the active ingredient (i.e. compound of the invention) per square metre body surface area. However, in a preferred use of the present invention a compound of the invention is administered to a subject in need thereof in an amount ranging from about 20 mg to about 120 mg/m², preferably ranging from about 20 mg to about 100 mg/m². The duration of therapy with a compound of the invention will vary, depending on the severity of the disorder being treated and the condition and idiosyncratic response of each individual patient. In one preferred embodiment of a prophylactic or therapeutic use, between about 20 mg to about 120 mg/m² of the compound is parenterally administered to an adult per day, depending on the severity of the disorder.

As is known in the art, the pharmaceutically effective amount of a given composition will also depend on the administration route. In general the required amount will be higher, if the administration is through the gastrointestinal tract, and lower if the route of administration is parenteral, e.g., intravenous. Typically, a compound of the invention will be administered in ranges of about 30 mg to about 200 mg/m², preferably about 50 mg to about 120 mg/m², if oral administration is used and in ranges of about 20 mg to about 100 mg/m², if parenteral administration is used.

### Applications of the compounds having the formula (I)

The compounds having the formula (I) are particularly useful as a tubulin polymerization inhibitor and/or an antimitotic cytotoxin. This is because their structures ensure a disposition of substituent groups on the molecular periphery which is appropriate to ensure binding to their protein target, and because the isoquinoline biaryl structure of the molecular interior ensures stability of the compound.

The compounds having the formula (I) are also used in the treatment, amelioration or prevention of a disorder selected from a neoplastic disorder, atherosclerosis, psoriasis, restenosis, idiopathic pulmonary fibrosis, scleroderma, wet age-related macular degeneration, and cirrhosis of the liver.

Preferably, the disorder is a neoplastic disorder, in particular cancer. The cancer is not particularly limited, preferably the cancer is selected from leukemias including acute myeloid leukemia, myelodysplastic syndrome, fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon carcinoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilms' tumor, cervical cancer, testicular tumor, lung carcinoma, small cell lung carcinoma, bladder carcinoma, epithelial carcinoma, glioma, astrocytoma, medulloblastoma, craniopharyngioma, ependymoma, pinealoma, hemangioblastoma, acoustic neuroma, oligodendroglioma, meningioma, melanoma, neuroblastoma, and retinoblastoma.

The compounds having the formula (I) can be used in combination with one or more other medicaments. The type of the other medicaments is not particularly limited and will depend on the disorder to be treated. Preferably the other medicament will be a further medicament which is to be used to treat the same disorder from which the compound of the invention is administered, e.g., a neoplastic disorder such as cancer.

Various modifications and variations of the invention will be apparent to those skilled in the art without departing from the scope of the invention. Although the invention has been described in connection with specific preferred embodiments, it should be understood that the invention as claimed should not be unduly limited to such specific embodiments. Indeed, various modifications of the described modes for carrying out the invention which are obvious to those skilled in the relevant fields are intended to be covered by the present invention.

The following examples are merely illustrative of the present invention and should not be construed to limit the scope of the invention as indicated by the appended claims in any way.

### EXAMPLES

The invention will be more fully understood by reference to the following examples. They should not, however, be construed as limiting the scope of the invention.

### Abbreviations

- AcOH:: acetic acid
- Bn:: benzyl
- CDCl₃:: deuterated chloroform
- DAPI:: 4',6-diamidino-2-phenylindole
- DCM:: dichloromethane
- DMSO:: dimethylsulfoxide
- EB3-YFP:: end-binding protein 3 fused to yellow fluorescent protein
- EI:: electron ionization
- ESI:: electron spray ionization
- EtOAc:: ethyl acetate
- Hz:: Hertz
- HCI:: hydrochloric acid
- HeLa:: *Henrietta Lacks,* human cervical cancer cell line
- HL60:: Human promyelocytic leukemia cells, human cell line
- HRMS:: High-resolution MS
- EC₅₀:: half maximal effective concentration
- *J*:: coupling constant
- MS:: mass spectrometry
- MHz:: megahertz
- MT:: microtubule
- TMP:: 2,2,6,6-tetramethylpiperidino
- µg:: microgram
- µL:: microliter
- µM:: micromoles per liter
- nM:: nanomoles per liter
- mg:: milligram
- mL:: milliliter
- mmol:: millimole
- MTT:: 3-(4,5-dimethylthiazol-2-yl)-2,5-diphenyltetrazolium bromide
- NMR:: nuclear magnetic resonance
- THF:: tetrahydrofuran
- PBS:: phosphate buffered saline
- Pd(PPh₃)₄:: tetrakis(triphenylphosphine)palladium
- PhMe:: toluene
- RP:: reverse phase
- TLC:: thin layer chromatography
- Ts:: *p*-toluenesulfonyl
- TFA:: 2,2,2-trifluoroacetic acid

### General Experimental Conditions

Unless stated otherwise, (1) all reactions and characterisations were performed with unpurified, undried, non-degassed solvents and reagents, used as obtained, under closed air atmosphere without special precautions; (2) "hexanes" used for chromatography was distilled from commercial crude iso-hexane fraction on rotavap; (3) "column" and "chromatography" refer to flash column chromatography, performed on Merck silica gel Si-60 (40-63 µm); (4) procedures and yields are unoptimised; (5) yields refer to isolated chromatographically and spectroscopically pure materials, corrected for residual solvent content.

NMR: Standard NMR characterisation was by ¹H- and ¹³C-NMR spectra. Unless otherwise stated, a 400 MHz spectrometer used by default (400 MHz & 100 MHz for ¹H and ¹³C respectively) at 300K. Chemical shifts (δ) are reported in ppm calibrated to residual non-perdeuterated solvent as an internal reference.

Mass Spectra: Unit mass measurements were performed on an AGILENT 1200 SL coupled LC-MS system with ESI mode ionisation, with binary eluent mixtures of water:acetonitrile, with the water containing formic acid. HRMS was carried out by the Zentrale Analytik of the LMU, Munich using ESI or EI ionisation as specified.

### Synthesis Examples

### Examples A-1 and A-2

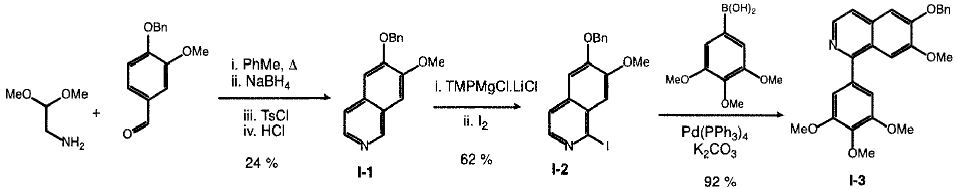

### 1-1: 6-benzyloxy-7-methoxyisoquinoline

4-Benzyloxy-3-methoxybenzaldehyde (4.99 g, 20.6 mmol) was dissolved in toluene (50 mL) and aminoacetaldehyde dimethyl acetal (2.38 g, 22.7 mmol) was added. Using a Dean-Stark apparatus the reaction mixture was heated to reflux for 16 h. After cooling to room temperature the volatiles were evaporated and the crude redissolved in MeOH (100 mL). The mixture was cooled to 0 °C and NaBH₄ (1.56 g, 41.2 mmol) was added portionwise over 30 min, then the reaction mixture was warmed to room temperature and stirred for 4 h. The volatiles were evaporated and the crude taken up in water (100 mL) then extracted with DCM (3 × 100 mL). The combined organic layers were dried over Na₂SO₄, filtered and concentrated. The crude product was redissolved in DCM (100 mL) and NaOH (1.40 g, 35.0 mmol) and tetrabutylammonium hydrogensulfate (0.490 g, 1.44 mmol) were added. After stirring for 10 min at room temperature a solution of p-toluenesulfonyl chloride (4.71 g, 24.7 mmol) in DCM (60 mL) was added dropwise over 1 h. The reaction mixture was stirred for an additional hour. Water (100 mL) was added, the phases were separated, the organic phase washed with water (2 x 100 mL) and brine (100 mL), dried over Na₂SO₄ and concentrated. The resulting crude product was dissolved in 1,4-dioxane (150 mL), aq. 6 M HCI (30 mL) was added and the reaction mixture heated to reflux for 16 h. After cooling to room temperature, the solution was poured into water (150 mL) and washed with diethyl ether (2 x 100 mL). Using a 6 M NaOH-solution the aqueous phase was adjusted to pH > 9 and extracted with DCM (3 x 150 mL). The combined organic layers were dried over Na₂SO₄ and concentrated. The resulting crude product was purified by flash column chromatography (100 % EtOAc) giving **I-1** (1.294 g, 24 %) as a white solid (HPLC-purity > 99 %; melting point 140 - 141 °C).
**¹H NMR (400 MHz, CDCl₃):** *δ* (ppm) = 9.04 (s, 1H, 1-H), 8.36 (d, J = 5.6 Hz, 1H, 3-H), 7.51 - 7.47 (m, 2H, 2'-, 6'-H), 7.44 (d, J = 5.6 Hz, 1H, 4-H), 7.43 - 7.38 (m, 2H, 3'-, 5'-H), 7.36 - 7.31 (m, 1H, 4'-H), 7.21 (s, 1H, 8-H), 7.10 (s, 1H, 5-H), 5.29 (s, 2H, CH₂), 4.03 (s, 3H, OCH₃).
**¹³C NMR (101 MHz, CDCl₃):** *δ* (ppm) = 152.3 (C-6), 150.8 (C-7), 150.1 (C-1), 142.1 (C-3), 136.2 (C-1'), 132.5 (C-4a), 128.9 (2C, C-3',-5'), 128.3 (C-4'), 127.4 (2C, C-2',-6'), 125.0 (C-8a), 119.4 (C-4), 106.5 (C-5), 105.7 (C-8), 70.9 (CH₂), 56.2 (OCH₃).
**HRMS (EI):** m/z = 265.1110 (calculated for C₁₇H₁₅NO₂: 265.1103).

### I-2: 6-benzyloxy-1-iodo-7-methoxyisoquinoline

To a solution of **I-1** (0.531 g, 2.00 mmol) in dry THF (8 mL) was slowly added TMPMgCl·LiCl (1.0 M in THF/toluene; 3.0 mL, 3.00 mmol) dropwise at room temperature. After 4 h the reaction mixture was cooled to 0 °C, a solution of iodine (0.761 g, 3.00 mmol) in dry THF (3 mL) was added dropwise and the resulting mixture stirred while warming to room temperature over 1 h. Sat. aq. NH₄Cl (4 mL) and sat. aq. Na₂S₂O₃ (4 mL) were added and the organic materials extracted using DCM (3 x 50 mL). The combined organic layers were dried over Na₂SO₄ and concentrated *in vacuo.* The resulting crude product was purified by flash column chromatography (DCM/EtOAc = 5:1) giving **I-2** (0.482 g, 62 %) as a brown solid (HPLC-purity: 98 %; melting point 139 - 140 °C).
**¹H NMR (400 MHz, CDCl₃):** *δ* (ppm) = 8.09 (d, J = 5.4 Hz, 1H, 3-H), 7.50 - 7.46 (m, 2H, 2'-,6'-H), 7.43 - 7.34 (m, 5H, 3'-,4'-,5'-, 4-, 8-H), 7.04 (s, 1H, 5-H), 5.30 (s, 2H, CH₂), 4.08 (s, 3H, OCH₃).
**¹³C NMR (101 MHz, CDCl₃):** *δ* (ppm) = 152.7 (C-6), 152.0 (C-7), 142.0 (C-3), 135.9 (C-1'), 132.5 (C-1), 128.9 (2C, C-3', -5'), 128.5 (C-4'), 128.3 (C-8a), 127.5 (2C, C-2', -6'), 124.9 (C-4a), 120.3 (C-4), 111.4 (C-8), 106.9 (C-5), 71.1 (CH₂), 56.4 (OCH₃).
**HRMS (EI):** m/z = 391.0070 (calculated for C₁₇H₁₄INO₂: 391.0069).

### I-3: 6-benzyloxy-7-methoxy-1-(3,4,5-trimethoxyphenyl)isoquinoline

To a solution of **I-2** (0.391 g, 1.00 mmol) in THF (6 mL) 3,4,5-trimethoxyphenylboronic acid (0.254 g, 1.20 mmol) was added. After addition of Pd(PPh₃)₄ (0.059 g, 0.050 mmol) and an aq. K₂CO₃-solution (1.0 M in H₂O; 3.0 mL, 3.00 mmol) the reaction mixture was stirred in a pressure tube under nitrogen at 90 °C for 16 h. After cooling to room temperature the solution was poured into water (50 mL) and extracted with EtOAc (3 × 50 mL). Die combined organic layers were dried over Na₂SO₄ and concentrated *in vacuo.* The crude product was purified by flash column chromatography (EtOAc/DCM = 2 : 1) giving **I-3** (0.395 g, 92 %) as a yellow solid (HPLC-purity: 98 %; melting point 152 - 153 °C).
**¹H NMR (400 MHz, CD₂Cl₂):** *δ* (ppm) = 8.40 (d, J = 5.5 Hz, 1H, 3-H), 7.52 - 7.48 (m, 3H, 2"-, 6"-, 4-H), 7.47 (s, 1H, 8-H), 7.46 - 7.41 (m, 2H, 3"-, 5"-H), 7.40 - 7.35 (m, 1H, 4"-H), 7.23 (s, 1H, 5-H), 6.93 (s, 2H, 2'-, 6'-H), 5.24 (s, 2H, CH₂), 3.88 (s, 6H, 3'-, 5'-OCH₃), 3.87 (s, 3H, 4'-OCH₃), 3.86 (s, 3H, 7-OCH₃).
**¹³C NMR (101 MHz, CD₂Cl₂):** *δ* (ppm) = 158.5 (C-1), 153.8 (2C, C-3', -5'), 152.4 (C-6), 151.0 (C-7), 141.7 (C-3), 138.8 (C-4'), 136.7 (C-1"), 136.2 (C-1'), 134.2 (C-4a), 129.2 (2C, C-3", - 5"), 128.9 (C-4"), 128.5 (2C, C-2", -6"), 123.0 (C-8a), 119.2 (C-4), 107.5 (2C, C-2', -6'), 107.0 (C-5), 106.3 (C-8), 71.3 (CH₂), 61.1 (4'-OCH₃), 56.7 (2C, 3'-, 5'-OCH₃), 56.4 (7-OCH₃). **HRMS (EI):** m/z (%) = 431.1729 (calculated for C₂₆H₂₅NO₅: 431.1733).

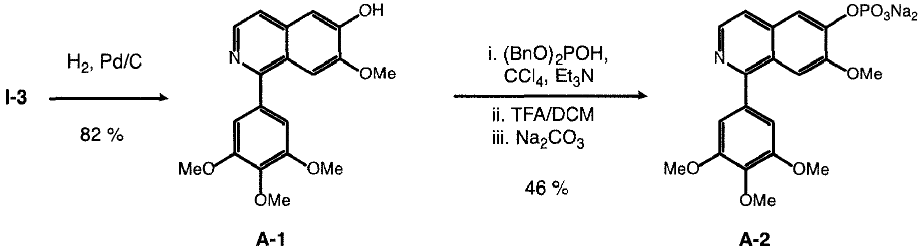

### A-1: 7-methoxy-1-(3,4,5-trimethoxyphenyl)isoquinolin-6-ol

To a solution of **I-3** (0.341 g, 0.790 mmol) in methanol (30 mL) was added 10 %-Pd/C (0.100 g). The reaction mixture was stirred under an atmosphere of hydrogen at room temperature for 24 h. The solution was filtered through a pad of celite and the filtrate concentrated *in vacuo* giving **A-1** (0.220 g, 82 %) as a white solid (HPLC-purity: >99 %; melting point 223 - 224 °C).
**¹H NMR (400 MHz, (CD₃)₂SO):** *δ* (ppm) = 10.29 (s, 1H, OH), 8.31 (d, J = 5.6 Hz, 1H, 3-H), 7.53 (d, J = 5.6 Hz, 1H, 4-H), 7.44 (s, 1H, 8-H), 7.22 (s, 1H, 5-H), 6.99 (s, 2H, 2'-, 6'-H), 3.84 (s, 6H, 3'-, 5'-OCH3), 3.82 (s, 3H, 7-OCH3), 3.76 (s, 3H, 4'-OCH3).
**¹³C NMR (101 MHz, (CD₃)₂SO):** *δ* (ppm) = 156.9 (C-1), 152.7 (2C, C-3', -5'), 150.8 (C-6), 149.6 (C-7), 140.4 (C-3), 137.6 (C-4'), 135.3 (C-1'), 133.6 (C-4a), 121.1 (C-8a), 118.1 (C-4), 108.5 (C-5), 107.0 (2C, C-2', -6'), 105.3 (C-8), 60.1 (4'-OCH3), 56.0 (2C, 3'-, 5'-OCH3), 55.4 (7-OCH3).
**HRMS (EI):** m/z = 341.1256 (calculated for C₁₉H₁₉NO₅: 341.1263).

### A-2: disodium 7-methoxy-1-(3,4,5-trimethoxyphenyl)isoquinolin-6-yl phosphate

To a solution of **A-1** (3.30 g, 9.67 mmol), Et₃N (2.69 mL, 19.3 mmol), and CCl₄ (4.66 mL, 48.3 mmol) in dry CH₃CN (30 mL) at 0°C, was added dropwise a solution of dibenzyl phosphite (3.55 g, 13.5 mmol) in dry CH₃CN (40.5 mL), and the resulting mixture stirred while warming to room temperature over 2 h. The volatiles were evaporated and the crude partitioned between water and ethyl acetate; the organic layer was dried over Na₂SO₄ and filtered. The filtrate was concentrated, then dissolved in a 1:1 TFA:DCM mixture (75 mL) and stirred overnight, capped under nitrogen. The volatiles were evaporated and phosphate buffer (50 mL) was added (pH ∼7). The aqueous phase was washed with 1:1 hexanes:EtOAc (3 x 50 mL). The aqueous layer was then adjusted to pH 4-5 and the organic materials extracted using EtOAc (3 x 50 mL). The organic layer was dried over Na₂SO₄, filtered and concentrated. The residue was dissolved in MeOH (50 mL) and NaHCO₃ (1.79 g, 21.3 mmol) was added. The reaction was stirred for one hour. The solvent was removed *in vacuo,* the crude product redissolved in MeOH (30 mL) and RP-silica was added (pH ∼7). After evaporation of the volatiles, reverse-phase column chromatography using 1 % acetonitrile in water gave the disodium salt **A-2** (2.10 g, 46 %) as a yellow solid (HPLC-purity: 98 %).
**¹H NMR (500 MHz, MeOD):** *δ* (ppm) = 8.23 (d, *J* = 5.7 Hz, 1H, 3-H), 8.14 (s, 1H, 5-H), 7.64 (d, *J* = 5.7 Hz, 1H, 4-H), 7.39 (s, 1H, 8-H), 6.95 (s, 2H, 2'-H and 6'-H), 3.89 (s, 6H, 3'-OCH₃ and 5'-OCH₃), 3.87 (s, 3H, 4'-OCH₃), 3.83 (s, 3H, 7-OCH₃).
**¹³C NMR (126 MHz, MeOD):** *δ* (ppm) = 158.9 (C-1), 154.5 (C-3' and C-5'), 153.4 (C-7), 150.3 (C-6), 140.1 (C-3), 139.6 (C-4'), 136.6 (C-1'), 135.7 (C-4a), 124.1 (C-8a), 121.0 (C-4), 115.1 (C-5), 108.3 (C-2' and C-6'), 106.4 (C-8), 61.2 (4'-OCH₃), 56.8 (3'-OCH₃ and 5'-OCH₃), 56.2 (7-OCH₃).
**HRMS (ESI+):** m/z = 422.0996 [M+H]⁺ (calculated for C₁₉H₂₁NO₈P⁺: 422.0999).

### Examples 3 and 4:

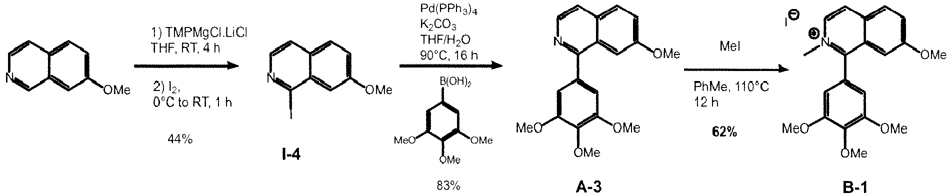

### I-4: 1-iodo-7-methoxyisoquinoline

TMPMgCl·LiCl (1.0 M in THF/toluene; 5.65 mL, 5.65 mmol) was added dropwise at room temperature to a solution of 7-methoxyisoquinoline (600 mg, 3.77 mmol) in dry THF (11 mL). After 4 h the reaction mixture was cooled to 0 °C, a solution of iodine (1.43 g, 5.65 mmol) in dry THF (6.65 mL) was added dropwise and the resulting mixture stirred while warming to room temperature over 1 h. Sat. aq. NH₄Cl (6 mL) and sat. aq. Na₂S₂O₃ (6 mL) were added and the **organic** materials extracted using DCM (3 x 50 mL). The combined organic layers were dried over Na₂SO₄ and concentrated in vacuo. The resulting crude product was purified by flash column chromatography (10 % EtOAc in hexanes) giving **I-4** (469 mg, 44 %) as a yellowish solid.
**¹H NMR (500 MHz, CDCl₃):** *δ* (ppm) = 8.16 (d, *J* = 5.5 Hz, 1H), 7.67 - 7.63 (m, 1H), 7.51 (dd, *J* = 5.5, 0.7 Hz, 1H), 7.38 - 7.34 (m, 2H), 4.00 (s, 3H).
**¹³C NMR (126 MHz, CDCl₃):** *δ* (ppm) = 160.0, 141.5, 133.5, 131.8, 129.1, 125.9, 124.3, 121.2, 110.6, 55.8.
**HRMS (ESI+):** m/z = 285.9722 [M+H]⁺ (calculated for C₁₀H₉INO⁺: 285.9723).

### A-3: 7-methoxy-1-(3,4,5-trimethoxyphenyl)isoquinoline

3,4,5-Trimethoxyphenylboronic acid (0.254 g, 1.20 mmol) was added to a solution of 1-iodo-7-methoxyisoquinoline (400 mg, 1.40 mmol) in THF (8 mL). After addition of Pd(PPh₃)₄ (0.081 g, 0.070 mmol) and an aq. K₂CO₃-solution (1.0 M in H₂O; 4.2 mL, 4.2 mmol) the reaction mixture was stirred under nitrogen at 90 °C for 16 h. After cooling to room temperature the solution was poured into water (30 mL) and extracted with EtOAc (3 × 50 mL). The combined organic layers were dried over Na₂SO₄ and concentrated *in vacuo.* The crude product was purified by flash column chromatography (50 % EtOAc in hexanes) giving **A-3** (377 mg, 83 %) as a yellow solid.
**¹H NMR (500 MHz, CDCl₃):** *δ* (ppm) = 8.49 (d, *J* = 5.6 Hz, 1H), 7.80 (d, *J* = 8.9 Hz, 1H), 7.59 (dd, *J* = 5.6, 0.9 Hz, 1H), 7.46 (d, *J* = 2.5 Hz, 1H), 7.37 (dd, *J* = 8.9, 2.5 Hz, 1H), 6.95 (s, 2H), 3.94 (s, 3H), 3.91 (s, 6H), 3.83 (s, 3H).
**¹³C NMR (126 MHz, CDCl₃):** *δ* (ppm) = 159.0, 158.5, 153.4, 140.4, 138.5, 135.4, 132.7, 128.8, 127.9, 123.3, 119.9, 107.1, 105.2, 61.1, 56.4, 55.6.
**HRMS (ESI+):** m/z = 326.1385 [M+H]⁺ (calculated for C₁₉H₂₀NO₄⁺: 326.1389).

### B-1: 7-methoxy-2-methyl-1-(3,4,5-trimethoxyphenyl)isoquinolin-2-ium iodide

**A-3** (200 mg, 0.615 mmol) was placed in a pressure tube and dissolved in toluene (3 mL). Mel (57.4 µL, 0.922 mmol) was added and the reaction was heated to reflux for 7 hours, after which time the reaction mixture was allowed to cool to room temperature. The resulting precipitate was collected, washed with toluene and dried under reduced pressure to give **B-1** (224 mg, 78 %) as a yellow solid.
**¹H NMR (500 MHz, DMSO-*d*₆):** *δ* (ppm) = 8.80 (d, *J* = 6.7 Hz, 1H), 8.57 (d, *J* = 6.7 Hz, 1H), 8.35 (d, *J* = 9.0 Hz, 1H), 7.92 (dd, *J* = 9.0, 2.5 Hz, 1H), 7.07 (s, 2H), 6.95 (d, *J* = 2.5 Hz, 1H), 4.14 (s, 3H), 3.83 (s, 3H), 3.82 (s, 6H), 3.80 (s, 3H).
**¹³C NMR (126 MHz, DMSO-*d*₆):** *δ* (ppm) = 160.3, 156.5, 153.5, 139.0, 134.8, 133.2, 129.7, 129.4, 128.3, 124.7, 124.7, 107.2, 106.7, 60.3, 56.3, 55.9, 47.4.
**HRMS (ESI+):** m/z = 340.1541 [M]⁺ (calculated for C₂₀H₂₂NO₄⁺: 340.1543).

### Biological Testing

**1) Antiproliferative effects** were determined by MTT assay in HeLa and HL60 cell lines in culture using standard protocols (M. Borowiak et al.; Cell 2015, 162, 403-411) after 48 h incubation. IC₅₀ values of representative compound **A-1** are 100 nM in HL60 and 240 nM in HeLa cell lines. The IC₅₀ value of representative compound **A-2** is 130 nM in HeLa cell line. The IC₅₀ value of representative compound **A-3** is 280 nM in HL60 cell line.
**2) Inhibition of tubulin polymerisation dynamics *in cellulo*** was assayed by confocal video microscopy in live HeLa cells transfected to express EB3-YFP fusion protein, a fluorescent marker that selectively labels the growing tips of microtubules (MTs). This manifests as moving "comets" when MTs are polymerising normally; the comets stop and disappear upon inhibition of MT polymerisation (M. Borowiak et al.; Cell 2015, 162, 403-411). After applying **A-2** (0.24 µM) the comets stopped and vanished within 0.5 minutes of treatment, showing that **A-2** causes potent inhibition of tubulin polymerisation dynamics (Figure 1).
**3) Microtubule perturbation *in cellulo*** was assayed by immunofluorescence staining of α-tubulin according to standard protocols (M. Borowiak et al.; Cell 2015, 162, 403-411), in HeLa cells following 24 h incubation with **A-2** (administered in PBS) or **A-3** (administered with 0.25% DMSO cosolvent) (Figure 2). The pronounced destruction of the microtubule network observed for both compounds is only expected for the most potent members of the CDI class.
**4) Cell cycle analysis** of representative compound **A-2** was performed by flow cytometric analysis of HeLa cells incubated with **A-2** for 24 h and then stained for DNA content with propidium iodide following standard protocols (M. Borowiak et al.; Cell 2015, 162, 403-411). Cells were analysed into cell cycle populations in subG1, G1, S and G2/M phase according to DNA content. Results showed extensive cell cycle arrest leading to accumulation of cells in the G2/M phase of the cell cycle (Figure 3), which is expected for potent CDIs displaying antimitotic cytotoxic effects by interrupting mitotic spindle reorganisation and completion of mitosis.
**5) Selected parameters of *in vivo* drug metabolism and interaction** of representative compound **A-1** were assayed *in vitro,* and safe dosing levels established *in vivo,* to evaluate key elements of the druglike suitability of the compounds of the invention.
   (a) Metabolic stability of **A-1** in the presence of mouse and human liver microsomes, at 2 uM, was assayed over a 40 minute timecourse at 37°C as is standard practice (D.-J. Hwang et al.; ACS Med. Chem. Lett. 2015, 6, 993-997). In human microsomes, the degradation rate of **A-1** was identical to reference compound imipramine (31% over 40 minutes), under conditions where second reference compound caffeine was <10% metabolised. In mouse microsomes, imipramine was >90% degraded after 30 minutes but **A-1** had only 33% degradation over 40 minutes, demonstrating excellent stability. The result may be very favourably compared to that of compound 2 from Hwang et al., which had half-life 39 min in human microsomes and only 2 min in mouse microsomes under the same assay conditions (see Table 2 in D.-J. Hwang et al.; ACS Med. Chem. Lett. 2015, 6, 993-997). Therefore we conclude that compounds of the invention are substantially metabolically stable, suiting them for further pharmacological evaluation.
   (b) Inhibition of five major human cytochrome P450s was assessed. CYP2D6, CYP2C9, CYP3A4, CYP2C19, CYP1A2 were chosen; a 10 minute preincubation then 25 minute competitive reaction with fluorogenic CYP substrates was performed [60 min reaction in the case of CYP2C19]. **A-1** was used at 10 µM and the assays run under standard conditions. CYP2D6, CYP2C9 and CYP3A4 showed only from 13-19% inhibition; CYP1A2 showed zero inhibition; and CYP2C19 showed an apparent enhancement of 50% activity. Therefore we conclude that no significant inhibition of these CYPs is caused by compounds of the invention, and thus that the compounds of the invention may escape typical major drug-drug interaction pathways, suiting them for further pharmacological evaluation.
   (c) LogD at pH 7.4 was assessed experimentally by octanol/water shake test for **A-1** and found to be 2.18, with reference to control compound mebendazole showing LogD of 2.9 under the same conditions. Therefore we conclude that the LogD of the active compounds of the invention is within a range considered optimum (1-3) for general *in vivo* use, suiting them for further pharmacological evaluation.
   (d) Safe dosing parameters of **A-1**'s fully water-soluble prodrug **A-2** (as disodium salt) were determined *in vivo* in male and female Balb/c mice by standard methods following i.v. and i.p. compound administration. Single dosage of 50 mg/kg i.v. was tolerated by all mice tested, and single dosage of 31.5 mg/kg i.p. was tolerated by all mice tested. Repeated dosage studies were conducted at dosage of 25 mg/kg (delivered by injection at 100 µL total volume, ie. 12.5 mM compound concentration in phosphate buffered saline at injection) both i.v. and i.p. with 3 administrations at 48-hour intervals. Note that this requirement for a high injection concentration in aqueous media underscores the emphasis placed on requiring compounds of the invention to be water soluble in order to have practical application *in vivo.* All mice tested in the repeated dosing trials tolerated compound administration.

## Claims

1. A compound having the formula (I) 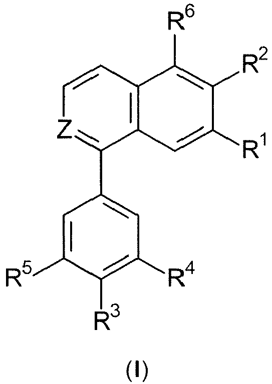 wherein
**Z** is selected from N and N⁺-R^{a}X;
**X** is a counterion;
**R¹** is selected from -C₁₋₄-alkyl, -O-(C₁₋₄-alkyl), -S-(C₁₋₄-alkyl), and -N(R^{b})(R^{c});
**R²** is selected from -H, -OH, -SH, -NH(R^{d}), -halogen, -CH₃, and -OPO₃(R^{e})(R^{f}), or R¹ and R² can be linked together to form -OCH₂O-,
**R³** is selected from -C₁₋₄-alkyl, -OH, -O-(C₁₋₄-alkyl), -SH, -S-(C₁₋₄-alkyl), -halogen, and -OPO₃(R^{e})(R^{f}), wherein -C₁₋₄-alkyl, -O-(C₁₋₄-alkyl) and -S-(C₁₋₄-alkyl) can be optionally substituted by a substituent A;
**R⁴** is selected from -C₁₋₄-alkyl, -O-(C₁₋₄-alkyl), and -S-(C₁₋₄-alkyl), wherein -C₁₋₄-alkyl, -O-(C₁₋₄-alkyl) and -S-(C₁₋₄-alkyl) can be optionally substituted by a substituent A;
or R³ and R⁴ can be linked together to form -OCH₂O- or -OCH₂CH₂O-;
**R⁵** is selected from -C₁₋₄-alkyl, -O-(C₁₋₄-alkyl), and -S-(C₁₋₄-alkyl), wherein -C₁₋₄-alkyl, -O-(C₁₋₄-alkyl) and -S-(C₁₋₄-alkyl) can be optionally substituted by a substituent A;
**R⁶** is selected from -H, -OH, -SH, -NH(R^{d}), -halogen, -CH₃, and -OPO₃(R^{e})(R^{f});
**R^{a}** is selected from -H and -C₁₋₄-alkyl;
**R^{b}** is selected from -C₁₋₄-alkyl and -proteinogenic amino acids;
**R^{c}** is selected from -H and -C₁₋₄-alkyl;
**R^{d}** is selected from -H, -C₁₋₄-alkyl, and -proteinogenic amino acids;
**R^{e}** and **R^{f}** are independently selected from H, ammonium, alkali ion or alkaline earth metal ion;
**R^{g}** is selected from -H and -C₁₋₄-alkyl;
**R^{h}** is selected from -H and -C₁₋₄-alkyl; and
**A** is selected from -OH, -SH, -N(R^{g})(R^{h}), azetidinyl, and -halogen; wherein azetidinyl is attached to the -C₁₋₄-alkyl, -O-(C₁₋₄-alkyl), or -S-(C₁₋₄-alkyl) group bearing it via the N atom;
provided that no more than one of the groups **R¹, R², R³, R⁴,** and **R⁵** may simultaneously be -C₁₋₄-alkyl;
or a pharmaceutically acceptable salt, solvate, or ester thereof;
provided that the following compounds as well as pharmaceutically acceptable salts and solvates thereof are excluded:

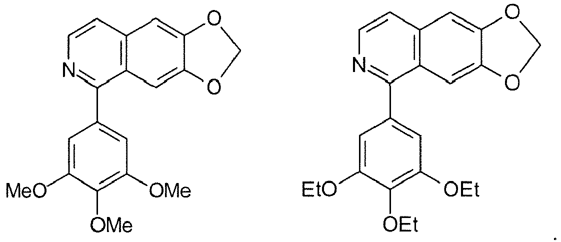

2. The compound according to claim 1, wherein R¹ and R² are linked together to form -OCH₂O-.

3. The compound according to claim 1, wherein **R¹** is selected from -C₁₋₄-alkyl, -O-(C₁₋₄-alkyl), and -S-(C₁₋₄-alkyl); preferably wherein **R¹** is -O-(C₁₋₄-alkyl).

4. The compound according to claim 1 or 3, wherein **R²** is selected from -H, -OH, -NH(R^{d}), and -OPO₃(R^{e})(R^{f}), preferably wherein **R²** is selected from -NH(R^{d}) and -OPO₃(R^{e})(R^{f}).

5. The compound according to any one of claims 1 to 4, wherein **R³** is selected from -C₁₋₄-alkyl, -OH, -O-(C₁₋₄-alkyl), and -OPO₃(R^{e})(R^{f}), preferably wherein **R³** is -O-(C₁₋₄-alkyl).

6. The compound according to any one of claims 1 to 5, wherein **R⁴** is selected from -C₁₋₄-alkyl and -O-(C₁₋₄-alkyl) and/or **R⁵** is selected from -C₁₋₄-alkyl and -O-(C₁₋₄-alkyl).

7. The compound according to any one of claims 1 to 6, wherein **R⁶** is selected from -H, -OH, -NH(R^{d}), and -OPO₃(R^{e})(R^{f}), preferably wherein **R⁶** is selected from -OH and -OPO₃(R^{e})(R^{f}).

8. The compound according to any one of claims 1 to 7, wherein **Z** is N.

9. The compound according to any one of claims 1 to 7, wherein **Z** is N⁺-R^{a}X, preferably wherein **R^{a}** is -C₁₋₄-alkyl.

10. A pharmaceutical composition comprising a compound having the formula (I), or a pharmaceutically acceptable salt, solvate, or ester thereof, and optionally a pharmaceutically acceptable carrier, 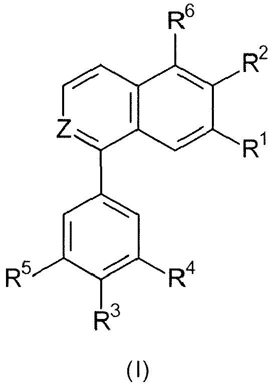 wherein
**Z** is selected from N and N⁺-R^{a}X;
**X** is a counterion;
**R¹** is selected from -C₁₋₄-alkyl, -O-(C₁₋₄-alkyl), -S-(C₁₋₄-alkyl), and -N(R^{b})(R^{c});
**R²** is selected from -H, -OH, -SH, -NH(R^{d}), -halogen, -CH₃, and -OPO₃(R^{e})(R^{f}), or R¹ and R² can be linked together to form -OCH₂O-;
**R³** is selected from -C₁₋₄-alkyl, -OH, -O-(C₁₋₄-alkyl), -SH, -S-(C₁₋₄-alkyl), -halogen, and -OPO₃(R^{e})(R^{f}), wherein -C₁₋₄-alkyl, -O-(C₁₋₄-alkyl) and -S-(C₁₋₄-alkyl) can be optionally substituted by a substituent A;
**R⁴** is selected from -C₁₋₄-alkyl, -O-(C₁₋₄-alkyl), and -S-(C₁₋₄-alkyl), wherein -C₁₋₄-alkyl, -O-(C₁₋₄-alkyl) and -S-(C₁₋₄-alkyl) can be optionally substituted by a substituent A;
or R³ and R⁴ can be linked together to form -OCH₂O- or -OCH₂CH₂O-;
**R⁵** is selected from -C₁₋₄-alkyl, -O-(C₁₋₄-alkyl), and -S-(C₁₋₄-alkyl), wherein -C₁₋₄-alkyl, -O-(C₁₋₄-alkyl) and -S-(C₁₋₄-alkyl) can be optionally substituted by a substituent A;
**R⁶** is selected from -H, -OH, -SH, -NH(R^{d}), -halogen, -CH₃, and -OPO₃(R^{e})(R^{f});
**R^{a}** is selected from -H and -C₁₋₄-alkyl;
**R^{b}** is selected from -C₁₋₄-alkyl and -proteinogenic amino acids;
**R^{c}** is selected from -H and -C₁₋₄-alkyl;
**R^{d}** is selected from -H, -C₁₋₄-alkyl, and -proteinogenic amino acids;
**R^{e}** and **R^{f}** are independently selected from H, ammonium, alkali ion or alkaline earth metal ion;
**R^{g}** is selected from -H and C₁₋₄-alkyl;
**R^{h}** is selected from -H and C₁₋₄-alkyl; and
**A** is selected from -OH, -SH, -N(R^{g})(R^{h}), azetidinyl, and -halogen; wherein azetidinyl is attached to the -C₁₋₄-alkyl, -O-(C₁₋₄-alkyl), or -S-(C₁₋₄-alkyl) group bearing it via the N atom;
and provided that no more than one of the groups **R¹, R², R³, R⁴,** and **R⁵** may simultaneously be -C₁₋₄-alkyl,
provided that the following compounds as well as pharmaceutically acceptable salts and solvates thereof are excluded:

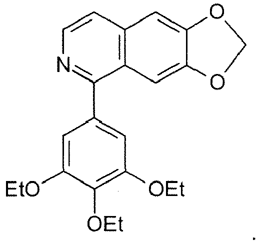

11. A compound having the formula (I), or a pharmaceutically acceptable salt, solvate, or ester thereof, for use as a tubulin polymerization inhibitor and/or an antimitotic cytotoxin, 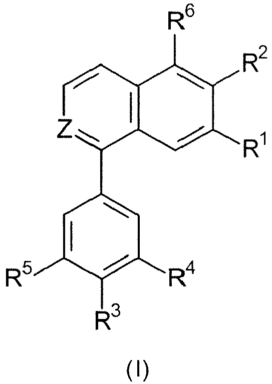 wherein
**Z** is selected from N and N⁺-R^{a}X;
**X** is a counterion;
**R¹** is selected from -C₁₋₄-alkyl, -O-(C₁₋₄-alkyl), -S-(C₁₋₄-alkyl), and -N(R^{b})(R^{c});
**R²** is selected from -H, -OH, -SH, -NH(R^{d}), -halogen, -CH₃, and -OPO₃(R^{e})(R^{f}), or R¹ and R² can be linked together to form -OCH₂O-;
**R³** is selected from -C₁₋₄-alkyl, -OH, -O-(C₁₋₄-alkyl), -SH, -S-(C₁₋₄-alkyl), -halogen, and -OPO₃(R^{e})(R^{f}), wherein -C₁₋₄-alkyl, -O-(C₁₋₄-alkyl) and -S-(C₁₋₄-alkyl) can be optionally substituted by a substituent A;
**R⁴** is selected from -C₁₋₄-alkyl, -O-(C₁₋₄-alkyl), and -S-(C₁₋₄-alkyl), wherein -C₁₋₄-alkyl, -O-(C₁₋₄-alkyl) and -S-(C₁₋₄-alkyl) can be optionally substituted by a substituent A; or R³ and R⁴ can be linked together to form -O-(C₁₋₂alkylene)-O-;
**R⁵** is selected from -C₁₋₄-alkyl, -O-(C₁₋₄-alkyl), and -S-(C₁₋₄-alkyl), wherein -C₁₋₄-alkyl, -O-(C₁₋₄-alkyl) and -S-(C₁₋₄-alkyl) can be optionally substituted by a substituent A;
**R⁶** is selected from -H, -OH, -SH, -NH(R^{d}), -halogen, -CH₃, and -OPO₃(R^{e})(R^{f});
**R^{a}** is selected from -H and -C₁₋₄-alkyl;
**R^{b}** is selected from -C₁₋₄-alkyl and -proteinogenic amino acids;
**R^{c}** is selected from -H and -C₁₋₄-alkyl;
**R^{d}** is selected from -H, -C₁₋₄-alkyl, and -proteinogenic amino acids;
**R^{e}** and **R^{f}** are independently selected from H, ammonium, alkali ion or alkaline earth metal ion;
**R^{g}** is selected from -H and C₁₋₄-alkyl;
**R^{h}** is selected from -H and C₁₋₄-alkyl; and
**A** is selected from -OH, -SH, -N(R^{g})(R^{h}), -azetidinyl, and -halogen; wherein azetidinyl is attached to the -C₁₋₄-alkyl, -O-(C₁₋₄-alkyl), or -S-(C₁₋₄-alkyl) group bearing it via the N atom,
and provided that no more than one of the groups **R¹, R², R³, R⁴,** and **R⁵** may simultaneously be -C₁₋₄-alkyl.

12. A compound having the formula (I), or a pharmaceutically acceptable salt, solvate, or ester thereof, for use in the treatment, amelioration or prevention of a disorder selected from a neoplastic disorder, atherosclerosis, psoriasis, restenosis, idiopathic pulmonary fibrosis, scleroderma, wet age-related macular degeneration, and cirrhosis of the liver, wherein the compound having the formula (I) is as defined in claim 11.

13. The compound for use according to claim 12, wherein the disorder is a neoplastic disorder, in particular cancer.

14. The compound for use according to claim 13, wherein the cancer is selected from leukemias including acute myeloid leukemia, myelodysplastic syndrome, fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon carcinoma, pancreatic cancer, breast cancer, ovarian cancer, prostate cancer, squamous cell carcinoma, basal cell carcinoma, adenocarcinoma, hepatocellular carcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilms' tumor, cervical cancer, testicular tumor, lung carcinoma, small cell lung carcinoma, non-small cell lung carcinoma, bladder carcinoma, epithelial carcinoma, glioblastoma, glioma, astrocytoma, medulloblastoma, craniopharyngioma, ependymoma, pinealoma, hemangioblastoma, acoustic neuroma, oligodendroglioma, meningioma, melanoma, neuroblastoma, and retinoblastoma.

15. The compound for use according to claim 13 or 14, wherein the compound is to be administered in combination with one or more other pharmaceutically active agents, which may include small molecule drugs (that are preferably cytotoxic chemotherapeutic drugs) that may be administered as nanoparticle formulations such as micelles, liposomes, nanoparticles including protein-bound formulations such as nanoparticular albumin-bound formulations, or virus-like particles, and/or which may include therapeutically active nanoparticles, such as antibodies, nanobodies, radiotherapeutic nanoparticles, and magnetotherapeutic nanoparticles.
